# EUROPEAN PATENT APPLICATION

(11) **EP 4 273 116 A1**
(43) Date of publication of application: **08.11.2023**
(21) Application number: 21912464.1
(22) Date of filing: 20.01.2021
(51) Int. Cl.: C07C 39/21, C07C 37/84, A61K 31/05, A61P 35/00, A61P 31/00, A61P 7/02, A61P 25/22, A61P 25/24, A61P 39/06, A61K 9/127, A61K 9/19, A61K 47/24, A61K 47/28

(54) **CRYSTAL FORM AND AMORPHOUS FORM OF HONOKIOL, AND PREPARATION METHOD THEREFOR**

(30) Priority: 31.12.2020 CN 202011620565
(71) Applicant: CHENGDU JINRUI FOUNDATION BIOTECH CO., LTD., Chengdu, Sichuan 610000 (CN)
(72) Inventor: YE, Haoyu, Chengdu, Sichuan 610000 (CN); ZHU, Tianmin, Chengdu, Sichuan 610000 (CN); QIU, Neng, Chengdu, Sichuan 610000 (CN)
(74) Representative: Dehns
(86) International application number: PCT/CN2021/072853
(87) International publication number: WO 2022/141684

(57) **Abstract**

Provided are a crystal form A, crystal form B, crystal form C and amorphous form of honokiol and preparation methods therefor. The method comprises: dissolving honokiol in n-heptane, standing at room temperature overnight, the separated crystal being the crystal form A of honokiol; heating the crystal form A of honokiol and then cooling to room temperature, the obtained solid being the crystal form B of honokiol; heating the crystal form A of honokiol to a molten state, and then placing the molten state crystal form at a quenching temperature, the separated crystal being the crystal form C of honokiol; and the oily matter obtained by adding the crystal form A of honokiol with DMSO/H₂O antisolvent system being the amorphous form of honokiol. The crystal form A, crystal form B, crystal form C of honokiol have good solubility, good stability, low hygroscopicity, long-term storage, good reproducibility and are suitable for drug development. The amorphous of honokiol has good solubility, and is suitable for the development of drug formulations.

## Description

### TECHNICAL FIELD

The present invention relates to the medical field, in particular to honokiol crystal forms and amorphous form, and preparation methods thereof.

### BACKGROUD OF THE INVENTION

Honokiol, with the chemical name of 3',5-diallyl-[1,1'-biphenyl]-2,4'-diol, has the following structural formula shown in formula (I):

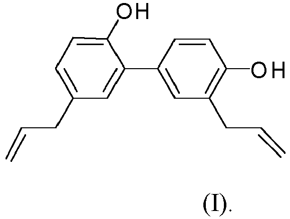

Honokiol, extracted from the bark of *Magnolia officinalis Rehd. et Wils*, is a small molecule compound with extensive biological activities, and the main biological activitiesQ thereof comprise antimicrobial, antioxidant, anxiolytic, antidepressant, and antithrombotic activities. Recently, more and more studies have shown that honokiol has a good anti-tumor activity, and an antitumor effect thereof is characterized by multiple targets, multiple effects and less toxic and side effects. Animal studies have shown that honokiol has a broad spectrum of anti-tumor activity against many kinds of rapidly growing tumors. It exerts anti-tumor effect by inducing tumor cell apoptosis, inhibiting migration and proliferation of tumor cells, and inhibiting tumor neovascularization.

The study of drug crystal form is one of the important contents in the study of new drugs. Therefore, it is of great significance to develop honokiol phase states with better physicochemical properties and bioavailability.

### SUMMARY

An object of the present invention is to provide honokiol crystal forms superior in solubility, stability, hygroscopicity, and the like.

In a first aspect of the present invention, a honokiol crystal form is provided, hereinafter referred to as honokiol crystal form A, wherein an X-ray powder diffraction pattern of honokiol crystal form A according to the present invention comprises characteristic peaks at 2theta values of 6.79°±0.2°, 9.10°±0.2°, 13.97°±0.2°, 14.97°±0.2° and 17.54°±0.2° under Cu-Kα radiation.

Further, the X-ray powder diffraction pattern of honokiol crystal form A according to the present invention comprises characteristic peaks at 2theta values of 6.79°±0.2°, 9.10°±0.2°, 13.97°±0.2°, 14.97°±0.2°, 17.54°±0.2°, 20.61°±0.2°, 22.08°±0.2° and 24.01°±0.2° under Cu-Kα radiation.

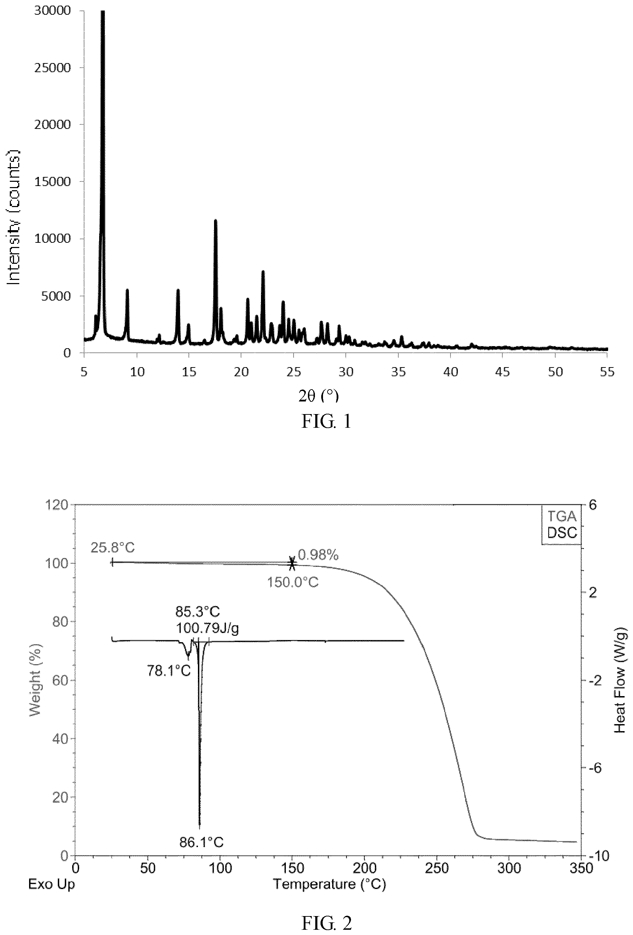

In a non-limiting manner, honokiol crystal form A according to the present invention has X-ray powder diffraction (XRPD) patterns as shown in FIG. 1, FIG. 3 to FIG. 9.

In a non-limiting manner, honokiol crystal form A according to the present invention has a thermogravimetric analysis (TGA)/differential scanning calorimetry (DSC) thermogram as shown in FIG. 2.

The present invention also provides a preparation method for honokiol crystal form A, comprising:
method I: preparing honokiol crystal form A by recrystallization
   dissolving honokiol in an organic solvent at 78-85°C to obtain a honokiol solution, rapidly pouring the honokiol solution into another container, standing at room temperature overnight, and centrifuging to collect a solid as honokiol crystal form A,
   wherein the organic solvent is n-heptane, petroleum ether, cyclohexane, n-hexane, toluene, DCM, DMSO, NMP, chloroform, methanol/water, or ethanol/water;
method II: preparing honokiol crystal form A by gas-solid infiltration
   placing honokiol in a container, then adding an organic solvent to another container, placing the opened container containing honokiol in the container containing the organic solvent, sealing and standing at room temperature (until the surface of honokiol is slightly wet), and centrifuging to collect a solid as honokiol crystal form A,
   wherein the organic solvent is ethyl acetate, isopropyl acetate, methyl tert-butyl ether, trichloromethane, N-methylpyrrolidone, ethanol or acetonitrile;
method III: preparing honokiol crystal form A by gas-liquid infiltration
   placing honokiol in a container, dissolving in a solvent and filtering to obtain a clear honokiol solution, adding an anti-solvent to another container, placing the opened container containing the clear honokiol solution in the container containing the anti-solvent, sealing and standing at room temperature, and centrifuging to collect a solid as honokiol crystal form A,
   wherein the solvent may be ethanol, tetrahydrofuran, trichloromethane, ethyl acetate or isopropanol, and the anti-solvent may be n-hexane or water;
method IV: preparing honokiol crystal form A by suspension stirring at room temperature
   placing honokiol in a container, adding an organic solvent to obtain a suspension, subjecting the obtained suspension to magnetic stirring at room temperature, and centrifuging to collect a solid as honokiol crystal form A,
   wherein the organic solvent may be methanol/water (94:6, 84:16, 69:31, 42:58), acetonitrile/water (1:9), acetone/water (1:9), or dichloromethane/n-hexane (1:9);
method V: preparing honokiol crystal form A by suspension stirring at 40-60°C
   placing honokiol in a container, adding an organic solvent to obtain a suspension, subjecting the obtained suspension to magnetic stirring at 40-60°C, and centrifuging to collect a solid as honokiol crystal form A,
   wherein the organic solvent may be n-heptane, Tween/n-heptane (1:9), trichloromethane/n-heptane (1:9), or isopropanol/water (1:9);
method VI: preparing honokiol crystal form A by stirring at temperature cycling
   placing honokiol in a container, adding an organic solvent to obtain a suspension, subjecting the obtained suspension to magnetic stirring at temperature cycling, and centrifuging to collect a solid as honokiol crystal form A,
   wherein the temperature cycling is composed of 2 cycles of 60°C→5°C, 0.1°C/min, and 5°C→60°C, 1.5°C/min, and preferably 2 cycles of 50°C→5°C, 0.1°C/min, and 5°C→50°C, 1.5°C/min,
   wherein the organic solvent may be n-heptane, ethanol/water (1:9), acetone/water (1:9), tetrahydrofuran/water (1:9), acetonitrile/water (1:9), ethyl acetate/n-heptane (1:9), methyl tert-butyl ether/n-hexane (1:9), or methyl isobutyl ketone/n-hexane (1:9);
method VII: preparing honokiol crystal form A by slow volatilization
   placing honokiol in a container, dissolving in an organic solvent, filtering and taking the filtrate, sealing the container containing the filtrate with a sealing membrane, making small holes on the sealing membrane, standing at room temperature to slowly volatilize, and centrifuging to collect a solid as honokiol crystal form A,
   wherein the organic solvent may be trichloromethane, dichloromethane, methanol, isopropanol, or methyl isobutyl ketone; and
method VIII: preparing honokiol crystal form A by anti-solvent addition
   placing honokiol in a container, dissolving in a solvent, adding an anti-solvent under magnetic stirring while adding dropwise, standing, and centrifuging to collect a solid as honokiol crystal form A,
   wherein the solvent may be ethanol, acetone, methyl isobutyl ketone, ethyl acetate, methyl tert-butyl ether, acetonitrile, dichloromethane or trichloromethane, and the anti-solvent may be n-heptane or water.

In a second aspect of the present invention, a honokiol crystal form is provided, hereinafter referred to as honokiol crystal form B, wherein an X-ray powder diffraction pattern of honokiol crystal form B according to the present invention comprises characteristic peaks at 2theta values of 6.15°±0.2°, 6.76°±0.2°, 8.96°±0.2° and 15.90°±0.2° under Cu-Kα radiation.

Further, the X-ray powder diffraction pattern of honokiol crystal form B according to the present invention comprises characteristic peaks at 2theta values of 6.15°±0.2°, 6.76°±0.2°, 8.96°±0.2°, 15.90°±0.2°, 17.49°±0.2° and 18.55°±0.2° under Cu-Kα radiation.

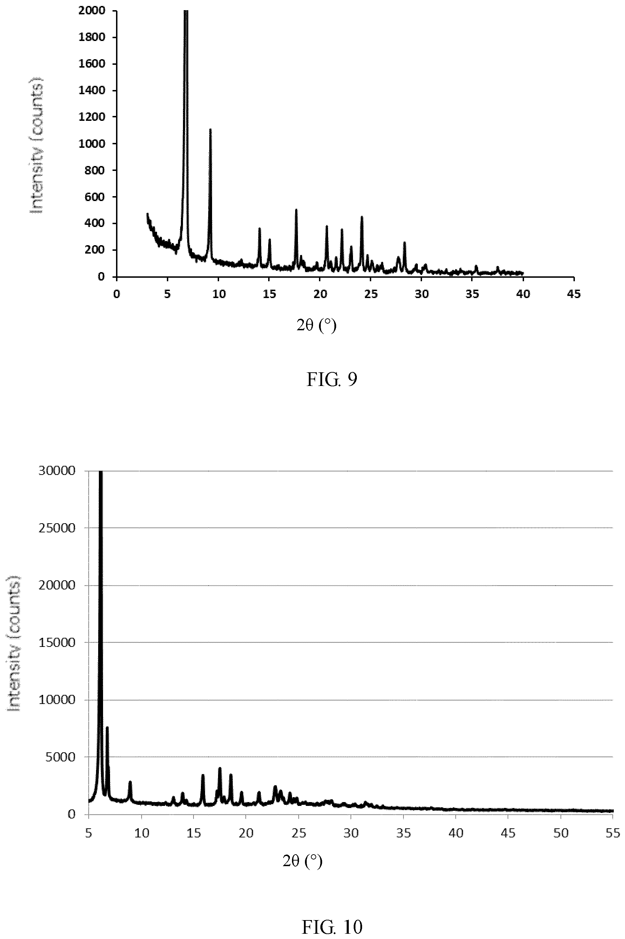

In a non-limiting manner, honokiol crystal form B according to the present invention has X-ray powder diffraction (XRPD) patterns as shown in FIG. 10, FIG. 12 to FIG. 15.

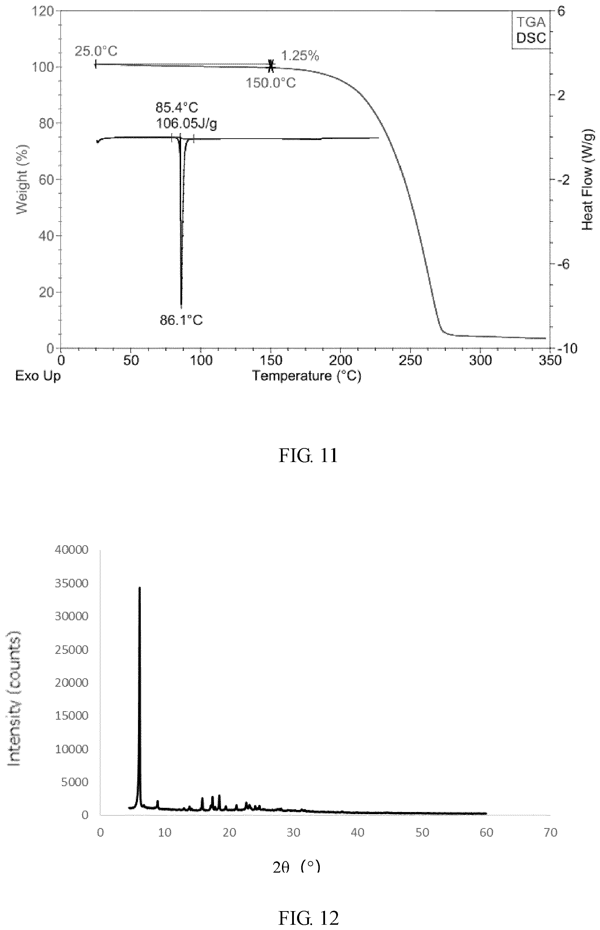

In a non-limiting manner, honokiol crystal form B according to the present invention has a thermogravimetric analysis (TGA)/differential scanning calorimetry (DSC) thermogram as shown in FIG. 11.

The present invention further provides a preparation method of honokiol crystal form B, comprising the following steps of
heating honokiol crystal form A to 80-82°C and then cooling to room temperature, thus obtaining a solid as honokiol crystal form B.

In a third aspect of the present invention, a honokiol crystal form is provided, hereinafter referred to as honokiol crystal form C, wherein an X-ray powder diffraction pattern of honokiol crystal form C according to the present invention comprises characteristic peaks at 2theta values of 14.04°±0.2°, 15.84°±0.2°, 17.42°±0.2° and 19.48°±0.2° under Cu-Kα radiation.

Further, the X-ray powder diffraction pattern of honokiol crystal form C according to the present invention comprises characteristic peaks at 2theta values of 14.04°±0.2°, 15.84°±0.2°, 17.42°±0.2°, 19.48°±0.2°, 21.26°±0.2°, 23.10°±0.2° and 24.06°±0.2° under Cu-Kα radiation.

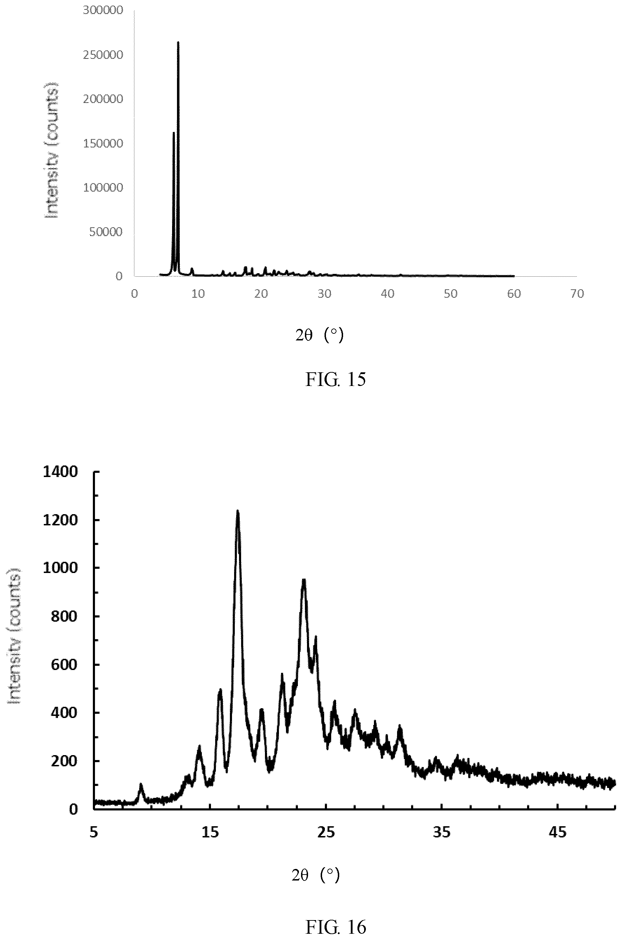

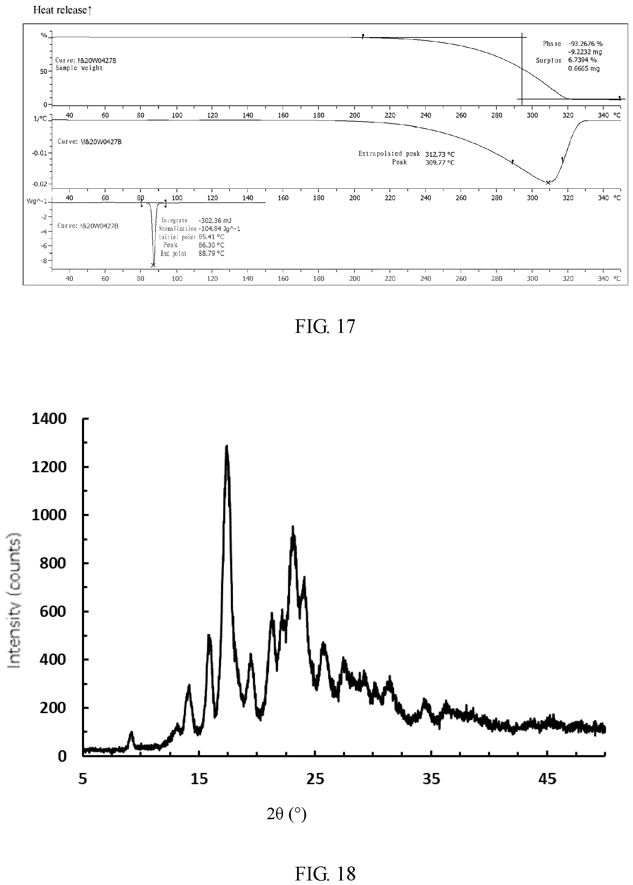

In a non-limiting manner, honokiol crystal form C according to the present invention has X-ray powder diffraction (XRPD) patterns as shown in FIG. 16 and FIG. 18.

In a non-limiting manner, the honokiol crystal form C according to the present invention has a thermogravimetric analysis (TGA)/differential scanning calorimetry (DSC) thermogram as shown in FIG. 17.

The present invention further provides a preparation method of honokiol crystal form C, comprising the following steps of
(1) heating honokiol crystal form A to a molten state at 83°C-100°C and stirring to fully mix until no obvious particles exist; and
(2) placing the melt obtained in step (1) rapidly at a quenching temperature -20°C to -196°C to separate out a crystal as honokiol crystal form C.

Preferably, in the preparation method of honokiol crystal form C, the quenching temperature of step (2) is -80°C to -196°C.

In a fourth aspect of the present invention, a honokiol amorphous form is provided, wherein the honokiol amorphous form according to the present invention has good solubility and is suitable for the development of drug formulations.

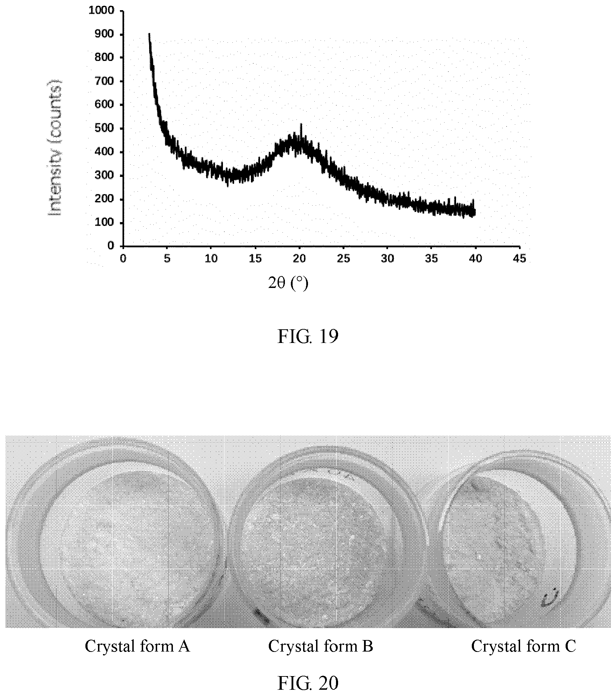

In a non-limiting manner, the honokiol amorphous form according to the present invention has X-ray powder diffraction (XRPD) pattern as shown in FIG. 19.

The present invention further provides a preparation method of the honokiol amorphous form, including the following steps of:
(1) dissolving honokiol crystal form A with DMSO;
(2) adding anti-solvent water to the solution obtained in step (1) and stirring; and
(3) centrifuging, discarding the supernatant, standing at room temperature, and thus obtaining an oily matter as the honokiol amorphous form.

Another object of the present invention is to provide a pharmaceutical composition or pharmaceutical preparation, comprising a therapeutically effective amount of honokiol crystal form A, honokiol crystal form B, honokiol crystal form C, or the honokiol amorphous form, and pharmaceutical excipients. The pharmaceutical composition or pharmaceutical preparation is prepared in a well-known way in the pharmaceutical field, and generally, a therapeutically effective amount of honokiol crystal form A, honokiol crystal form B, honokiol crystal form C or the honokiol amorphous form is mixed or contacted with one or more pharmaceutical excipients to prepare the pharmaceutical composition or the pharmaceutical preparation.

A further object of the present invention is to provide a honokiol nano-liposome lyophilized powder, comprising a therapeutically effective amount of honokiol crystal form A, honokiol crystal form B, honokiol crystal form C, or the honokiol amorphous form. The honokiol nano-liposome lyophilized powder is prepared by the following method: dissolving honokiol crystal form A, honokiol crystal form B, honokiol crystal form C or honokiol amorphous form (1 part), phospholipid (0.1-100 parts), phosphatidylethanolamine (0.01-100 parts) and cholesterol (0-100 parts) in absolute ethanol, injecting the solution that is completely dissolved into purified water, stirring, subjecting to rotary evaporation to remove ethanol, adding the freeze-dried excipient (1-50 parts), and freeze-drying to obtain the honokiol nano-liposome lyophilized powder. After reconstitution the lyophilized powder, a particle size of honokiol nano-liposome is 80-170 nm as determined by a laser particle size analyzer.

The phospholipid is selected from one or more of soybean phospholipid, egg yolk lecithin, hydrogenated soybean lecithin, hydrogenated egg yolk lecithin, phosphatidylcholine, phosphatidylethanolamine, phosphatidylglycerol and sodium salts thereof, phosphatidylinositol, cardiolipin, sphingomyelin or phosphatidylserine; phosphatidylethanolamine is selected from one or more of the Cultured phosphatidylethanolamine, soybean phosphatidylethanolamine, distearyl phosphatidylethanolamine-polyethylene glycol 600-20000, dipalmitoyl phosphatidylethanolamine-polyethylene glycol 600-20000, dioleoylphosphatidylethanolamine-polyethylene glycol 600-20000, stearoyloleoylphosphatidylethanolamine-polyethylene glycol 600-20000, stearoylimidylphosphatidylethanolamine-polyethylene glycol 600-20000, palmitoyloleoylphosphatidylethanolamine-polyethylene glycol 600-20000, dimyristoylphosphatidylethanolamine-polyethylene glycol 600-20000, dilaurylphosphatidylethanolamine-polyethylene glycol 600-20000, didecanoyl phosphatidylethanolamine-polyethylene glycol 600-20000, dioctanoylphosphatidylethanolamine-polyethylene glycol 600-20000 or dihexanoylphosphatidylethanolamine-polyethylene glycol 600-20000; and the excipients are selected from one or more of glucose, glycine, mannitol, inositol, sorbitol, sucrose, trehalose, lactose, galactose, glutamic acid, proline, lysine or alanine.

A further object of the present invention is to provide use of honokiol crystal form A, honokiol crystal form B, and honokiol crystal form C or the honokiol Amorphous form in the preparation of medicaments for treating tumors.

Yet another object of the present invention is to provide use of honokiol crystal form A, honokiol crystal form B, and honokiol crystal form C or the honokiol Amorphous form in the preparation of antimicrobial, antioxidant, anxiolytic, antidepressant or antithrombotic medicaments.

Honokiol crystal forms A, B and C of the present invention have the advantages of good solubility, good stability, low hygroscopicity, long-term storage and good reproducibility, and are suitable for drug development. The honokiol amorphous form of the present invention has good solubility and is suitable for drug preparation development.

### BRIEF DESCRIPTION OF THE DRAWINGS

FIG. 1 is an XRPD pattern of honokiol crystal form A in Example 1.
FIG. 2 is a TGA/DSC thermogram of honokiol crystal form A in Example 1.
FIG. 3 is an XRPD pattern of honokiol crystal form A in Example 2.
FIG. 4 is an XRPD pattern of honokiol crystal form A in Example 3.
FIG. 5 is an XRPD pattern of honokiol crystal form A in Example 4.
FIG. 6 is an XRPD pattern of honokiol crystal form A in Example 5.
FIG. 7 is an XRPD pattern of honokiol crystal form A in Example 6.
FIG. 8 is an XRPD pattern of honokiol crystal form A in Example 7.
FIG. 9 is an XRPD pattern of honokiol crystal form A in Example 8.
FIG. 10 is an XRPD pattern of honokiol crystal form B treated after cooling to the room temperature immediately after heating to 80°C in Example 9.
FIG. 11 is a TGA/DSC thermogram of honokiol crystal form B treated after cooling to the room temperature immediately after heating to 80°C in Example 9.
FIG. 12 is an XRPD pattern of honokiol crystal form B treated after heating to 80°C and holding for 1 hour and then cooling to the room temperature in Example 10.
FIG. 13 is an XRPD pattern of honokiol crystal form B treated after heating to 80°C and holding for 4 hours and then cooling to the room temperature in Example 11.
FIG. 14 is an XRPD pattern of honokiol crystal form B treated after heating to 80°C and holding for 8 hours and then cooling to the room temperature in Example 12.
FIG. 15 is an XRPD pattern of honokiol crystal form B treated after cooling to room temperature immediately after heating to 82°C in Example 13.
FIG. 16 is an XRPD pattern of honokiol crystal form C treated at -80°C in Example 14.
FIG. 17 is a TGA/DSC thermogram of honokiol crystal form C treated at -80°C in Example 14.
FIG. 18 is an XRPD pattern of honokiol crystal form C treated at -196°C in Example 15.
FIG. 19 is an XRPD pattern of a honokiol amorphous form prepared in Example 16.
FIG. 20 is photographs of honokiol crystal forms A, B and C after exposure to sunlight.
FIG. 21 is an HPLC pattern of honokiol crystal form A prior to a stress test.
FIG. 22 is an HPLC pattern of honokiol crystal form A after a high temperature test.
FIG. 23 is an HPLC pattern of honokiol crystal form A after a high humidity test.
FIG. 24 is an HPLC pattern of honokiol crystal form A after a high-light irradiation test.
FIG. 25 is an HPLC pattern of honokiol crystal form B prior to a stress test.
FIG. 26 is an HPLC pattern of honokiol crystal form B after a high temperature test.
FIG. 27 is an HPLC pattern of honokiol crystal form B after a high humidity test.
FIG. 28 is an HPLC pattern of honokiol crystal form B after a high-light irradiation test.
FIG. 29 is an HPLC pattern of honokiol crystal form C prior to a stress test.
FIG. 30 is an HPLC pattern of honokiol crystal form C after a high temperature test.
FIG. 31 is an HPLC pattern of honokiol crystal form C after a high humidity test.
FIG. 32 is an HPLC pattern of honokiol crystal form C after a high-light irradiation test.

### DESCRIPTION OF EXEMPLARY EMBODIMENTS

The following embodiments further illustrate the present invention, but the examples do not constitute a restriction or limitation on the scope of the present invention.

Reagents used in the examples of the present invention are conventional reagents in the field. Unless otherwise specified hereinafter, the reagents used in the present invention are well known in the field, but the reagents are described in the present invention in detail as much as possible.

Raw materials used in the examples and a preparation method of honokiol powder are detailed in Journal of Chromatography A, 1142 (2007) 115-122.

### Example 1: Preparing honokiol crystal form A by recrystallization with n-heptane

201.25 mg of honokiol powder was completely dissolved in n-heptane at 80°C, then the solution was poured to another beaker quickly, stood at room temperature overnight, thus obtaining a crystal as honokiol crystal form A, of which an XRPD pattern was shown in FIG. 1 and a TGA/DSC thermogram was shown in FIG. 2.

The corresponding values of 2theta values and intensities of honokiol crystal form A in this example were shown in Table 1:

**Table 1 Corresponding values of 2theta values and intensities of honokiol crystal form A**

| No. | 2theta | Intensity % |
|---|---|---|
| 1 | 6.0935 | 4.95 |
| 2 | 6.5328 | 16.41 |
| 3 | 6.7971 | 100 |
| 4 | 9.1031 | 9.68 |
| 5 | 12.1559 | 2.25 |
| 6 | 13.9719 | 9.78 |
| 7 | 14.9720 | 4.12 |
| 8 | 16.5075 | 1.41 |
| 9 | 17.5405 | 21.51 |
| 10 | 19.3106 | 1.6 |
| 11 | 19.5697 | 2.11 |
| 12 | 20.6195 | 8.33 |
| 13 | 20.9485 | 4.39 |
| 14 | 21.4800 | 5.3 |
| 15 | 22.0843 | 12.89 |
| 16 | 22.8333 | 4.1 |
| 17 | 22.9755 | 3.79 |
| 18 | 23.7021 | 3.91 |
| 19 | 24.0117 | 7.96 |
| 20 | 24.5432 | 4.73 |
| 21 | 25.0433 | 4.66 |
| 22 | 25.5378 | 3.06 |
| 23 | 25.8289 | 2.58 |
| 24 | 26.0495 | 3.15 |
| 25 | 27.2453 | 1.76 |
| 26 | 27.6398 | 4.35 |
| 27 | 28.2230 | 4.18 |
| 28 | 29.0779 | 1.63 |
| 29 | 29.3534 | 3.65 |
| 30 | 30.0155 | 2.14 |
| 31 | 30.3107 | 2 |
| 32 | 30.8450 | 1.41 |
| 33 | 31.5797 | 1.11 |
| 34 | 31.8736 | 1.12 |
| 35 | 33.7018 | 1.18 |
| 36 | 34.6063 | 1.37 |
| 37 | 35.3406 | 2.01 |
| 38 | 36.2867 | 0.91 |
| 39 | 37.4284 | 0.98 |
| 40 | 37.8896 | 0.88 |
| 41 | 38.7965 | 0.59 |
| 42 | 40.5974 | 0.49 |
| 43 | 42.0185 | 0.79 |
| 44 | 45.2149 | 0.3 |
| 45 | 49.5154 | 0.29 |
| 46 | 51.6076 | 0.21 |

### Example 2: preparing honokiol crystal form A by gas-solid infiltration

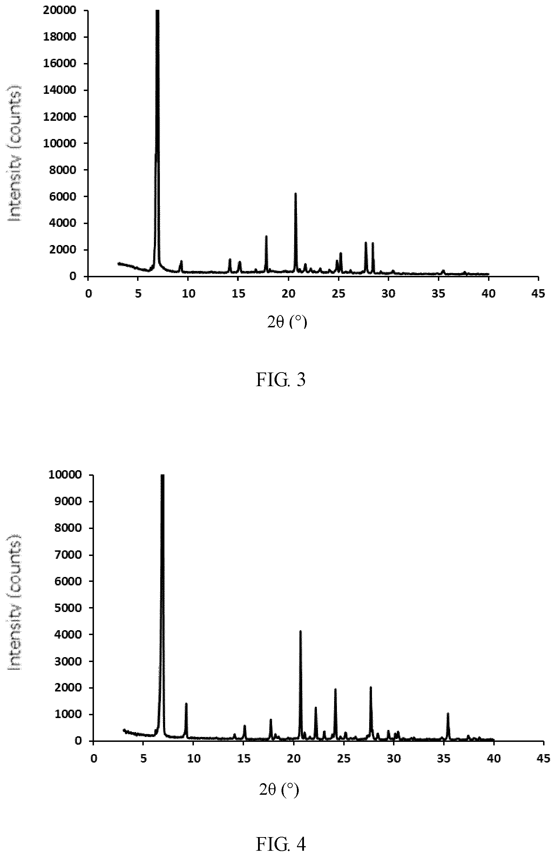

About 15 mg of honokiol powder was weighed into a 3 mL vial, about 3 mL of N-methylpyrrolidone was added into another 20 mL vial, the 3 mL opened vial was placed in the 20 mL vial and the 20 mL vial was sealed. The 20 mL vial stood at room temperature until the surface of the sample was slightly wet, the sample was centrifuged to collect a solid as honokiol crystal form A. The obtained solid was honokiol crystal form A as confirmed by an XRPD test, of which an XRPD pattern was shown in FIG. 3, and a TGA/DSC thermogram was consistent with the TGA/DSC thermogram of honokiol crystal form A prepared in Example 1.

### Example 3: preparing the honokiol crystal form A by gas-liquid infiltration

About 15 mg of honokiol powder was weighed into a 3 mL vial, dissolved in 0.5 mL of solvent isopropanol and filtered through a 0.45 µm PTFE membrane to obtain a clear solution. About 3 mL of anti-solvent water was added to another 20 mL vial, the 3 mL opened vial containing the clear solution was placed in the 20 mL vial, and then the 20 mL vial was sealed and stood at room temperature for 9 days. The sample was centrifuged to collect a solid as honokiol crystal form A. The obtained solid was honokiol crystal form A as confirmed by an XRPD test, of which an XRPD pattern was shown in FIG. 4, and a TGA/DSC thermogram was consistent with the TGA/DSC thermogram of honokiol crystal form A prepared in Example 1.

### Example 4: preparing honokiol crystal form A by suspension stirring at room temperature

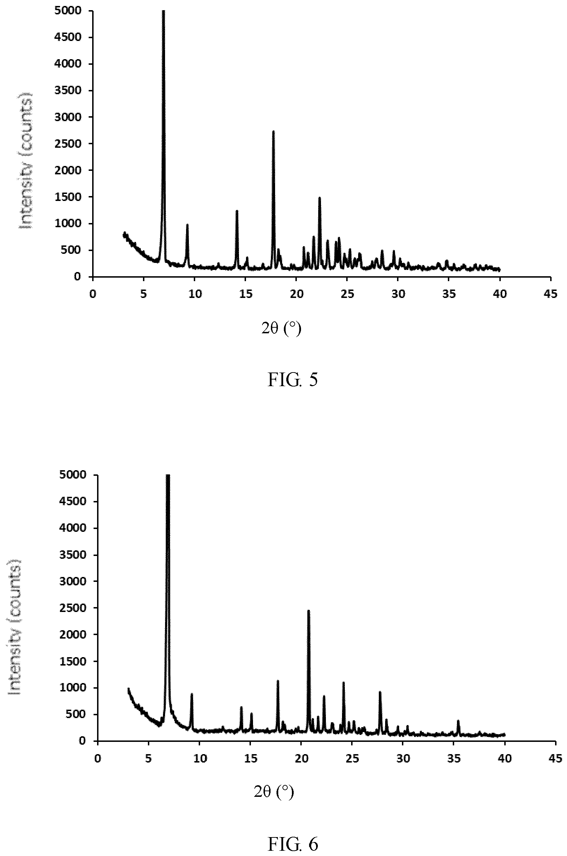

About 15 mg of honokiol powder was weighed into a 3 mL vial, and 0.3-0.5 mL of acetonitrile/water (1:9) was added to the vial. The obtained suspension was subjected to magnetic stirring at room temperature for 4 days, and then centrifuged to collect a solid as honokiol crystal form A. The obtained solid was honokiol crystal form A as confirmed by an XRPD test, of which an XRPD pattern was shown in FIG. 5, and a TGA/DSC thermogram was consistent with the TGA/DSC thermogram of honokiol crystal form A prepared in Example 1.

### Example 5: preparing honokiol crystal form A by suspension stirring at 50°C

About 15 mg of honokiol powder was weighed into a 3 mL vial, and 0.5 mL of trichloromethane/n-heptane (1:9) was added to the vial. The obtained suspension was subjected to magnetic stirring at 50° C and at 1,000 rpm for 4 days, and then centrifuged to collect a solid as honokiol crystal form A. The obtained solid was honokiol crystal form A as confirmed by an XRPD test, of which an XRPD pattern was shown in FIG. 6, and a TGA/DSC thermogram was consistent with the TGA/DSC thermogram of honokiol crystal form A prepared in Example 1.

### Example 6: preparing honokiol crystal form A by stirring at temperature cycling

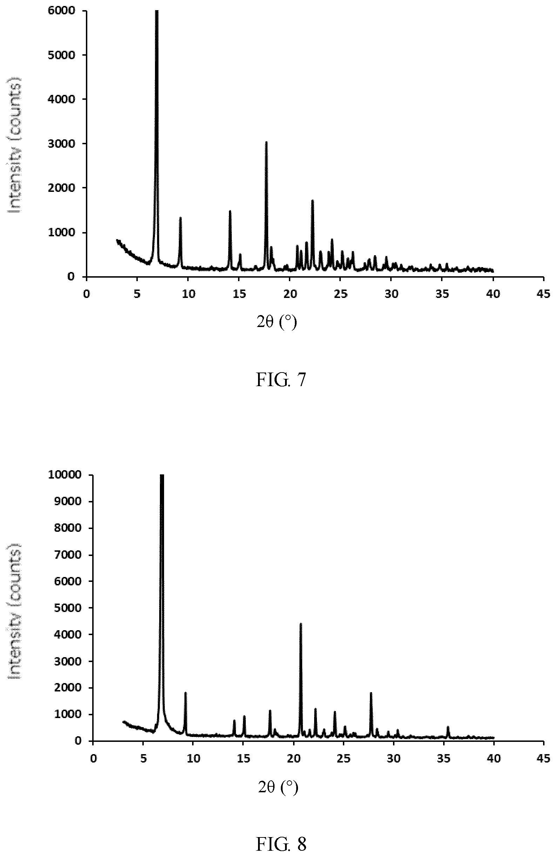

About 15 mg of honokiol powder was weighed into a 3 mL vial, and 0.5 mL of ethanol/water (1:9) was added to the vial. The obtained suspension was subjected to magnetic stirring at the temperature cycling (2 cycles: 50°C→5°C, 0.1°C/min, and 5°C→50°C, 1.5°C/min) and at 1,000 rpm for 16 hours, and then the sample was centrifuged to collect a solid as honokiol crystal form A. The obtained solid was honokiol crystal form A as confirmed by an XRPD test, of which an XRPD pattern was shown in FIG. 7, and a TGA/DSC thermogram was consistent with the TGA/DSC thermogram of the honokiol crystal form A prepared in Example 1.

### Example 7: preparing the honokiol crystal form A by slow volatilization

About 15 mg of honokiol powder was weighed into a 3 mL vial, dissolved in 1.0 mL of trichloromethane, filtered (with PTFE membrane with a pore size of 0.45 µm) and then the filtrate was taken. The vial containing clear solution was sealed with a sealing membrane, several small holes were made on the sealing membrane, the vial stood at room temperature to slowly volatilize to separate out a solid, and then the solid was collected as honokiol crystal form A by centrifuging. The obtained solid was honokiol crystal form A as confirmed by an XRPD test, of which an XRPD pattern was shown in FIG. 8, and a TGA/DSC thermogram was consistent with the TGA/DSC thermogram of honokiol crystal form A prepared in Example 1.

### Example 8: preparing honokiol crystal form A by anti-solvent addition

About 15 mg of honokiol powder was weighed into a 3 mL vial, dissolved in 0.5-1.0 mL of ethanol, n-heptane was added under magnetic stirring, the system was stirred while adding dropwise, stood overnight, and then centrifuged to collect a solid as honokiol crystal form A. The obtained solid was honokiol crystal form A as confirmed by an XRPD test, of which an XRPD pattern was shown in FIG. 9, and a TGA/DSC thermogram was consistent with the TGA/DSC thermogram of the honokiol crystal form A prepared in Example 1.

### Example 9: preparation of honokiol crystal form B

After heating 200.75 mg of honokiol crystal form A to 80°C, the sample was placed at room temperature immediately to obtain a solid as honokiol crystal form B, of which an XRPD spectrum was shown in FIG. 10 and a TGA/DSC thermogram was shown in FIG. 11.

The corresponding values of 2theta values and intensities of honokiol crystal form B in this example were shown in Table 2:

**Table 2 Corresponding values of 2theta values and intensities of honokiol crystal form B**

| No. | 2theta | Intensity % |
|---|---|---|
| 1 | 6.1504 | 100.00 |
| 2 | 6.7608 | 15.08 |
| 3 | 8.9653 | 4.93 |
| 4 | 13.0808 | 1.88 |
| 5 | 13.9526 | 2.77 |
| 6 | 14.3170 | 1.39 |
| 7 | 15.9024 | 6.34 |
| 8 | 17.2149 | 3.10 |
| 9 | 17.4955 | 7.62 |
| 10 | 17.9023 | 2.02 |
| 11 | 18.5502 | 6.26 |
| 12 | 19.5855 | 2.79 |
| 13 | 21.2438 | 2.88 |
| 14 | 22.7821 | 4.06 |
| 15 | 23.2199 | 2.99 |
| 16 | 23.5529 | 1.90 |
| 17 | 24.1781 | 2.76 |
| 18 | 24.5335 | 1.66 |
| 19 | 24.8318 | 1.84 |
| 20 | 26.7765 | 0.88 |
| 21 | 28.1861 | 1.33 |
| 22 | 29.3438 | 0.75 |
| 23 | 30.3149 | 0.58 |
| 24 | 31.3429 | 0.99 |
| 25 | 31.9348 | 0.60 |
| 26 | 37.6766 | 0.24 |

### Example 10: preparation of honokiol crystal form B

After heating 200.65 mg of honokiol crystal form A to 80°C and keeping the temperature for 1 hour, the sample was placed at room temperature immediately to obtain a solid as honokiol crystal form B, of which an XRPD spectrum was shown in FIG. 12 and a TGA/DSC thermogram was consistent with the TGA/DSC thermogram of honokiol crystal form B prepared in Example 9.

The corresponding values of 2theta values and intensities of honokiol crystal form B in this example were shown in Table 3:

**Table 3 Corresponding values of 2theta values and intensities of honokiol crystal form B**

| No. | 2theta | Intensity % |
|---|---|---|
| 1 | 6.1145 | 100 |
| 2 | 8.9191 | 4.83 |
| 3 | 13.0492 | 1.62 |
| 4 | 13.9199 | 2.4 |
| 5 | 14.2635 | 1.4 |
| 6 | 15.8646 | 6.33 |
| 7 | 17.1657 | 2.82 |
| 8 | 17.4554 | 6.9 |
| 9 | 17.8548 | 2.25 |
| 10 | 18.5209 | 7.66 |
| 11 | 19.5406 | 2.55 |
| 12 | 21.1944 | 2.98 |
| 13 | 22.733 | 4.21 |
| 14 | 23.1809 | 2.91 |
| 15 | 24.1315 | 2.66 |
| 16 | 24.7949 | 2.72 |
| 17 | 25.5231 | 1.15 |
| 18 | 28.0686 | 1.32 |
| 19 | 31.3283 | 1.03 |
| 20 | 37.6203 | 0.39 |

### Example 11: preparation of honokiol crystal form B

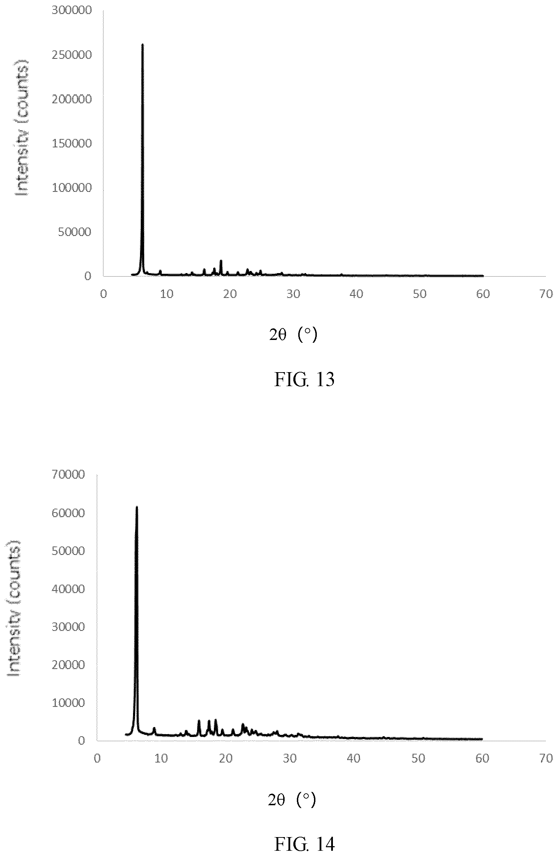

After heating 200.25 mg of honokiol crystal form A to 80°C and keeping the temperature for 4 hours, the sample was placed at room temperature immediately to obtain a solid as honokiol crystal form B, of which an XRPD spectrum was shown in FIG. 13 and a TGA/DSC thermogram was consistent with the TGA/DSC thermogram of honokiol crystal form B prepared in Example 9.

The corresponding values of 2theta values and intensities of honokiol crystal form B in this example were shown in Table 4:

**Table 4 Corresponding values of 2theta values and intensities of honokiol crystal form B**

| No. | 2theta | Intensity % |
|---|---|---|
| 1 | 6.1917 | 100 |
| 2 | 6.906 | 1.62 |
| 3 | 9.0082 | 2.3 |
| 4 | 12.3598 | 0.69 |
| 5 | 13.1118 | 0.79 |
| 6 | 13.9898 | 1.53 |
| 7 | 14.3439 | 0.65 |
| 8 | 15.9382 | 2.9 |
| 9 | 17.242 | 1.41 |
| 10 | 17.5357 | 3.32 |
| 11 | 17.9295 | 1.17 |
| 12 | 18.5849 | 6.77 |
| 13 | 19.6136 | 1.71 |
| 14 | 21.2758 | 1.66 |
| 15 | 22.796 | 2.94 |
| 16 | 23.3027 | 1.77 |
| 17 | 23.5706 | 0.92 |
| 18 | 24.211 | 1.3 |
| 19 | 24.5713 | 0.89 |
| 20 | 24.8569 | 2.3 |
| 21 | 25.7046 | 0.69 |
| 22 | 26.8102 | 0.51 |
| 23 | 27.5878 | 0.83 |
| 24 | 27.846 | 0.82 |
| 25 | 28.1965 | 1.3 |
| 26 | 29.2644 | 0.48 |
| 27 | 29.4753 | 0.48 |
| 28 | 30.4566 | 0.43 |
| 29 | 31.4005 | 0.71 |
| 30 | 31.9042 | 0.74 |
| 31 | 32.4824 | 0.32 |
| 32 | 33.0914 | 0.35 |
| 33 | 34.4144 | 0.23 |
| 34 | 37.6543 | 0.67 |
| 35 | 40.0415 | 0.16 |
| 36 | 41.6417 | 0.12 |
| 37 | 44.2058 | 0.11 |
| 38 | 44.6725 | 0.24 |
| 39 | 45.4141 | 0.1 |
| 40 | 46.396 | 0.08 |
| 41 | 49.6005 | 0.06 |
| 42 | 50.9781 | 0.15 |

### Example 12: preparation of honokiol crystal form B

After heating 201.25 mg of honokiol crystal form A to 80°C and keeping the temperature for 8 hours, the sample was placed at room temperature immediately to obtain a solid as honokiol crystal form B, of which an XRPD spectrum was shown in FIG. 14 and a TGA/DSC thermogram was consistent with the TGA/DSC thermogram of honokiol crystal form B prepared in Example 9.

The corresponding values of 2theta values and intensities of honokiol crystal form B in this example were shown in Table 5:

**Table 5 Corresponding values of 2theta values and intensities of honokiol crystal form B**

| No. | 2theta | Intensity % |
|---|---|---|
| 1 | 6.0859 | 89.22 |
| 2 | 6.2586 | 100 |
| 3 | 8.9298 | 4.87 |
| 4 | 12.3175 | 1.75 |
| 5 | 13.0913 | 2.39 |
| 6 | 13.9301 | 3.52 |
| 7 | 14.4509 | 1.84 |
| 8 | 15.8914 | 7.98 |
| 9 | 17.1827 | 3.65 |
| 10 | 17.4718 | 8.24 |
| 11 | 17.8736 | 3.23 |
| 12 | 18.4936 | 8.26 |
| 13 | 18.6688 | 5.88 |
| 14 | 19.5528 | 4.02 |
| 15 | 21.2134 | 4.17 |
| 16 | 22.7336 | 6.36 |
| 17 | 23.309 | 5.07 |
| 18 | 23.551 | 3.42 |
| 19 | 24.1664 | 4.01 |
| 20 | 24.491 | 2.68 |
| 21 | 24.7726 | 3.54 |
| 22 | 25.6034 | 2.24 |
| 23 | 26.7734 | 1.89 |
| 24 | 27.5516 | 2.85 |
| 25 | 28.1247 | 3.39 |
| 26 | 29.4852 | 1.76 |
| 27 | 30.349 | 1.66 |
| 28 | 31.3386 | 2.25 |
| 29 | 31.9246 | 1.77 |
| 30 | 33.0757 | 1.12 |
| 31 | 34.4329 | 0.88 |
| 32 | 37.5659 | 1.08 |
| 33 | 39.9031 | 0.49 |
| 34 | 41.5763 | 0.43 |
| 35 | 44.6686 | 0.66 |
| 36 | 45.4135 | 0.37 |
| 37 | 46.3251 | 0.3 |
| 38 | 47.6436 | 0.24 |
| 39 | 49.5829 | 0.18 |
| 40 | 50.9185 | 0.28 |

### Example 13: preparation of honokiol crystal form B

After heating 200.15 mg of honokiol crystal form A to 82°C, the sample was placed at room temperature immediately to obtain a solid as honokiol crystal form B, of which an XRPD spectrum was shown in FIG. 15 and a TGA/DSC thermogram was consistent with the TGA/DSC thermogram of honokiol crystal form B prepared in Example 9.

The corresponding values of 2theta values and intensities of honokiol crystal form B in this example were shown in Table 6:

**Table 6 Corresponding values of 2theta values and intensities of honokiol crystal form B**

| No. | 2theta | Intensity % |
|---|---|---|
| 1 | 6.1607 | 59.56 |
| 2 | 6.5174 | 4.42 |
| 3 | 6.8724 | 100 |
| 4 | 9.0088 | 2.83 |
| 5 | 9.1692 | 2.01 |
| 6 | 12.2552 | 0.15 |
| 7 | 13.0794 | 0.21 |
| 8 | 14.006 | 1.83 |
| 9 | 14.2782 | 0.18 |
| 10 | 15.0641 | 1.05 |
| 11 | 15.9119 | 1.2 |
| 12 | 16.5522 | 0.02 |
| 13 | 17.2202 | 0.8 |
| 14 | 17.4603 | 3.35 |
| 15 | 17.6241 | 3.47 |
| 16 | 17.9209 | 0.63 |
| 17 | 18.1087 | 0.95 |
| 18 | 18.5873 | 2.86 |
| 19 | 19.4273 | 0.48 |
| 20 | 19.6235 | 0.56 |
| 21 | 20.5742 | 2.29 |
| 22 | 20.7317 | 3.11 |
| 23 | 21.0086 | 0.44 |
| 24 | 21.2764 | 0.5 |
| 25 | 21.4974 | 0.46 |
| 26 | 22.0504 | 1.83 |
| 27 | 22.7693 | 1.11 |
| 28 | 23.2588 | 0.34 |
| 29 | 23.9398 | 0.89 |
| 30 | 24.1083 | 1.51 |
| 31 | 24.5834 | 0.41 |
| 32 | 24.8612 | 0.65 |
| 33 | 25.1094 | 0.98 |
| 34 | 25.5801 | 0.25 |
| 35 | 25.9375 | 0.42 |
| 36 | 27.6025 | 1.62 |
| 37 | 27.7528 | 1.62 |
| 38 | 28.0915 | 0.81 |
| 39 | 28.3164 | 0.99 |
| 40 | 29.3251 | 0.53 |
| 41 | 29.9711 | 0.25 |
| 42 | 30.4174 | 0.5 |
| 43 | 30.8365 | 0.03 |
| 44 | 31.3583 | 0.17 |
| 45 | 31.6421 | 0.29 |
| 46 | 31.9099 | 0.2 |
| 47 | 32.3041 | 0.06 |
| 48 | 33.1748 | 0.13 |
| 49 | 33.7905 | 0.13 |
| 50 | 34.4223 | 0.11 |
| 51 | 35.4539 | 0.53 |
| 52 | 36.381 | 0.12 |
| 53 | 37.4516 | 0.32 |
| 54 | 38.7171 | 0.05 |
| 55 | 39.5517 | 0.21 |
| 56 | 40.4904 | 0.03 |
| 57 | 42.0234 | 0.46 |
| 58 | 43.1904 | 0.04 |
| 59 | 44.0785 | 0.06 |
| 60 | 44.6456 | 0.13 |
| 61 | 45.3062 | 0.06 |
| 62 | 46.4142 | 0.09 |
| 63 | 47.6515 | 0.02 |
| 64 | 49.4697 | 0.18 |
| 65 | 51.7075 | 0.16 |
| 66 | 53.7499 | 0.04 |
| 67 | 57.3282 | 0.02 |

### Example 14: preparation of honokiol crystal form C

1,002.5 mg of honokiol crystal form A was heated to a molten state at 85°C in a crucible, then the crucible was quickly placed in a refrigerator at -80°C, stood overnight to obtain a solid as honokiol crystal form C, of which an XRPD spectrum was shown in FIG. 16 and a TGA/DSC thermogram was shown in FIG. 17.

The corresponding values of 2theta values and intensities of honokiol crystal form C in this example were shown in Table 7:

**Table 7 Corresponding values of 2theta values and intensities of honokiol crystal form C**

| No. | 2theta | Intensity % |
|---|---|---|
| 1 | 9.40 | 6.34 |
| 2 | 13.18 | 10.95 |
| 3 | 14.20 | 20.17 |
| 4 | 15.94 | 40.35 |
| 5 | 17.52 | 100 |
| 6 | 19.70 | 30.55 |
| 7 | 21.26 | 45.82 |
| 8 | 22.34 | 46.97 |
| 9 | 23.14 | 67.44 |
| 10 | 24.10 | 66.57 |
| 11 | 25.82 | 36.02 |
| 12 | 27.62 | 33.14 |
| 13 | 29.34 | 26.22 |
| 14 | 30.16 | 25.36 |
| 15 | 31.56 | 27.67 |
| 16 | 34.50 | 22.48 |

### Example 15: preparation of honokiol crystal form C

998.3 mg of honokiol crystal form A was heated to a molten state at 85°C in a crucible, and then the crucible was quickly placed in liquid nitrogen (-196°C) and stood overnight to obtain a solid as honokiol crystal form C, of which an XRPD spectrum was shown in FIG. 18 and a TGA/DSC thermogram was consistent with the TGA/DSC thermogram of honokiol crystal form C prepared in Example 14.

The corresponding values of 2theta values and intensities of honokiol crystal form C in this example were shown in Table 8:

**Table 8 Corresponding values of 2theta values and intensities of honokiol crystal form C**

| No. | 2theta | Intensity % |
|---|---|---|
| 1 | 9.20 | 7.76 |
| 2 | 13.14 | 10.56 |
| 3 | 14.18 | 22.98 |
| 4 | 15.88 | 39.13 |
| 5 | 17.42 | 100 |
| 6 | 19.48 | 32.76 |
| 7 | 21.28 | 46.12 |
| 8 | 22.18 | 47.20 |
| 9 | 23.10 | 74.07 |
| 10 | 24.10 | 57.76 |
| 11 | 25.70 | 36.80 |
| 12 | 27.62 | 30.90 |
| 13 | 29.28 | 26.86 |
| 14 | 30.18 | 23.60 |
| 15 | 31.44 | 24.53 |
| 16 | 34.54 | 18.17 |

### Example 16: preparation of honokiol amorphous form

About 15 mg of honokiol crystal form A was weighed into a 20 mL vial, dissolved in 0.5-1.0 mL of solvent DMSO, added with anti-solvent H₂O under magnetic stirring, and stirred while adding dropwise. The obtained solution was centrifuged, the supernatant was discarded, and then the remaining stood at room temperature to obtain an oily matter as the amorphous form, of which an XRPD pattern was shown in FIG. 19.

### Example 17: TGA/DSC tests for honokiol crystal forms A, B and C

Thermogravimetric Analysis (TGA): instrument manufacturer: METTLER-TOLEDO; instrument model: TGA/DSC2/1600; test conditions: taking an appropriate amount of trial-production sample under nitrogen atmosphere at 30-350°C, raising the temperature at a constant speed of 10°C/min, measuring changes in weight of the substance with the temperature, and plotting a weight-temperature change curve-TGA curve;

Differential Scanning Calorimeter (DSC): instrument manufacturer: METTLER-TOLEDO; instrument model: DSC3+/500; test conditions: taking an appropriate amount of trial-production sample under nitrogen atmosphere at 30-150°C, raising the temperature at a constant speed of 10°C/min, and determining a DSC curve of the sample; and

Heating honokiol crystal forms A, B and C from room temperature to 150°C respectively.

TGA results of honokiol crystal form A show a 1.0% weight loss, and DSC results show two endothermic signals at 78.1°C and 86.1°C (peak temperature) as shown in FIG. 2.

TGA results of honokiol crystal B show a 1.3% weight loss, and DSC results show an endothermic signal at 86.1 °C (peak temperature) as shown in FIG. 11.

TGA results of honokiol crystal form C show a 93.3% weight loss, and DSC results show an endothermic signal at 86.3°C (peak temperature) as shown in FIG. 17.

### Example 18: solubility tests for honokiol crystal forms A, B and C

Since a solubility of a drug directly affected dissolution and oral bioavailability of a pharmaceutical preparation, the inventor investigated the solubility of honokiol crystal forms A, B and C in PBS. Respective saturated solutions of honokiol crystal forms A, B and C were prepared, shaken at 37°C for 4 hours, centrifuged and then the supernatant was fed into a high performance liquid chromatograph. At the same time, a standard solution of honokiol (source: China Institutes for Food and Drug Control, Batch No.: 110730-201915, ID: EH2H-BMTW), and an external standard method was used for quantification. Thus it was measured that the solubility of honokiol crystal form A was 41.35 µg/ml, the solubility of honokiol crystal form B was 43.66 µg/ml, and the solubility of honokiol crystal form C was 48.12 µg/ml.

### Example 19: light stability tests for honokiol crystal forms A, B and C

Honokiol crystal forms A, B and C stood respectively in a small beaker in sunlight to investigate the stability of each of honokiol crystal forms A, B and C. Photographs of honokiol crystal forms A, B and C after exposure to sunlight were shown in FIG. 20.

### Example 20: stress tests for honokiol crystal forms A, B and C

Honokiol crystal forms A, B and C were treated under high temperature (60°C), high humidity (relative humidity 92.5%, 25°C) and high-light irradiation (illuminance 45,001 x ± 5,001 x), respectively, diluted with methanol to investigate the changes of related substances through testing with HPLC.

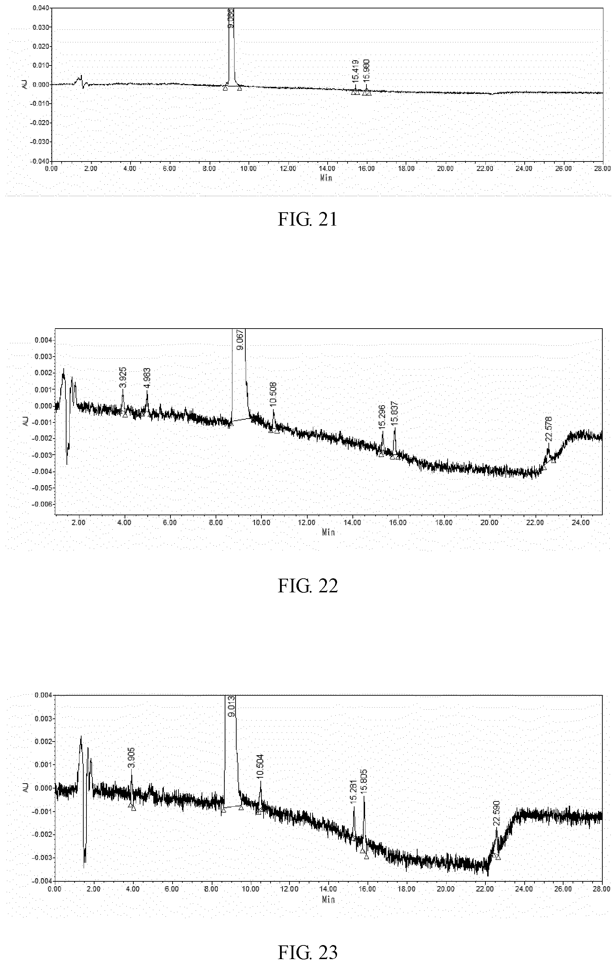

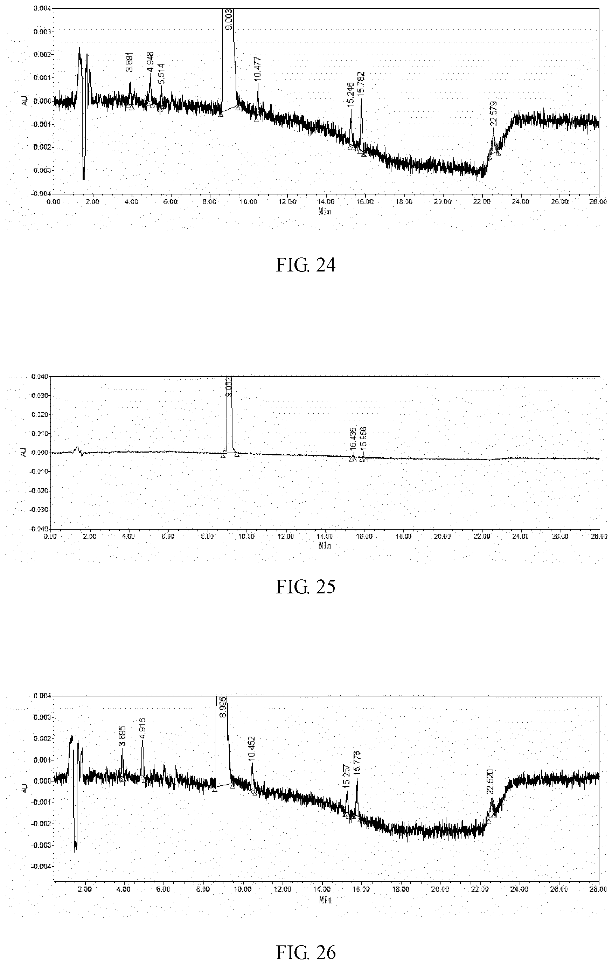

The purity of honokiol crystal form A was 99.83% before the stress test, and the purity of honokiol crystal form A was 99.63% after the high temperature test; the purity of honokiol crystal form A after the high humidity test was 99.67%; and the purity of honokiol crystal form A after the high-light irradiation test was 99.64%. HPLC patterns of honokiol crystal form A before the stress test were shown in FIG. 21, the HPLC patterns after the high temperature test were shown in FIG. 22, the HPLC patterns after the high humidity test were shown in FIG. 23, and the HPLC patterns after the high-light irradiation test were shown in FIG. 24.

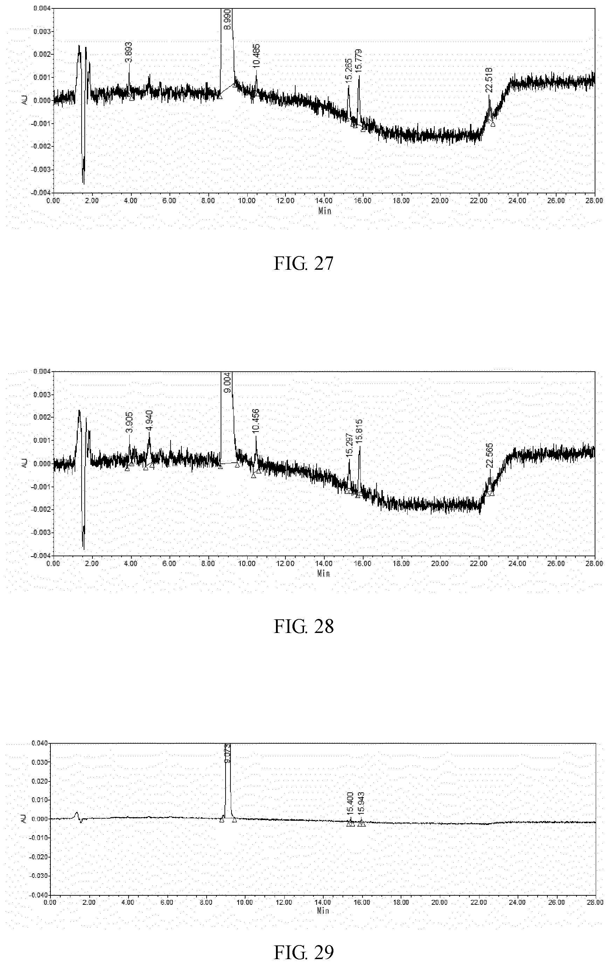

The purity of honokiol crystal form B was 99.88% before the stress test, and the purity of honokiol crystal form B was 99.70% after the high temperature test; the purity of honokiol crystal form B after the high humidity test was 99.70%; and the purity of honokiol crystal form B after the high-light irradiation test was 99.60%. HPLC patterns of honokiol crystal form B before the stress test were shown in FIG. 25, the HPLC patterns after the high temperature test were shown in FIG. 26, the HPLC patterns after the high humidity test were shown in FIG. 27, and the HPLC patterns after the high-light irradiation test were shown in FIG. 28.

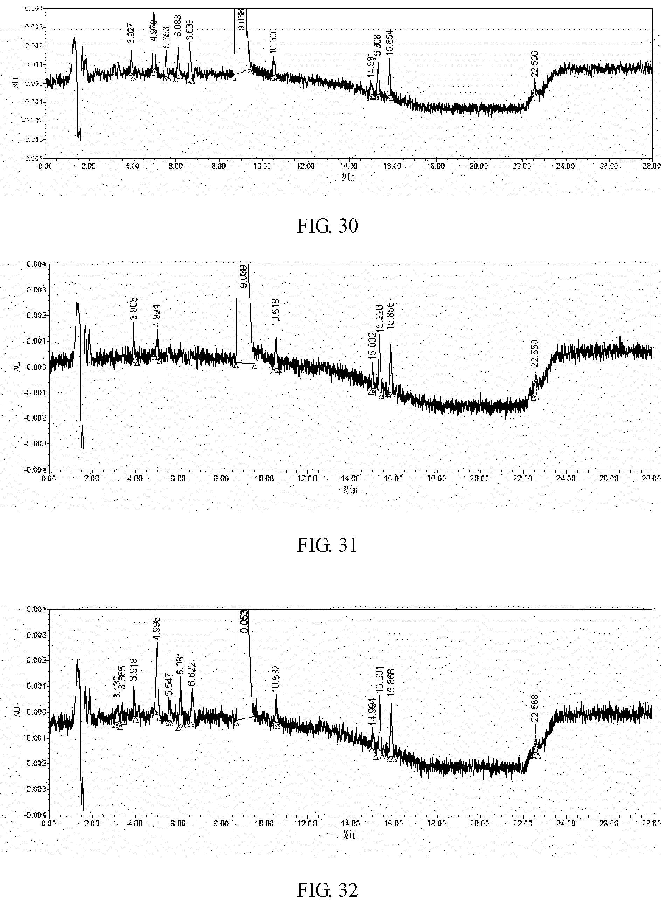

The purity of honokiol crystal form C was 99.86% before the stress test, and the purity of honokiol crystal form C was 99.36% after the high temperature test; the purity of honokiol crystal form C after the high humidity test was 99.59%; and the purity of honokiol crystal form C after the high-light irradiation test was 99.31%. HPLC patterns of honokiol crystal form C before the stress test were shown in FIG. 29, the HPLC patterns after the high temperature test were shown in FIG. 30, the HPLC patterns after the high humidity test were shown in FIG. 31, and the HPLC patterns after the high-light irradiation test were shown in FIG. 32.

### Example 21: preparation of honokiol nano-liposome lyophilized powder

All of honokiol crystal forms A, B and C and the amorphous form were used to prepare the honokiol nano-liposome lyophilized powder with the same preparation method. Taking honokiol crystal form B as an example, the preparation method was given below:
dissolving 50 mg of honokiol crystal form B, 500 mg of soybean phospholipid, 200 mg of cholesterol and 200 mg of Cultured phosphatidylethanolamine in 50 mL of absolute ethanol, dissolving completely, injecting the solution into 300 mL of purified water, stirring, subjecting to rotary evaporation to remove the ethanol, adding 800 mg of sucrose as the freeze-dried excipient, and freeze-drying to obtain the honokiol nano-liposome lyophilized powder. After reconstitution the lyophilized powder, a particle size of honokiol nano-liposome was 114 nm as determined by a laser particle size analyzer.

## Claims

1. A honokiol crystal form A, wherein an X-ray powder diffraction pattern of honokiol crystal form A comprises characteristic peaks at 2theta values of 6.79°±0.2°, 9.10°±0.2°, 13.97°±0.2°, 14.97°±0.2° and 17.54°±0.2° under Cu-Kα radiation.

2. The honokiol crystal form A according to claim 1, wherein the X-ray powder diffraction pattern of honokiol crystal form A comprises characteristic peaks at 2theta values of 6.79°±0.2°, 9.10°±0.2°, 13.97°±0.2°, 14.97°±0.2°, 17.54°±0.2°, 20.61°±0.2°, 22.08°±0.2° and 24.01°±0.2° under Cu-Kα radiation.

3. A preparation method of honokiol crystal form A according to claim 1 or 2, comprising:
method I: preparing honokiol crystal form A by recrystallization
dissolving honokiol in an organic solvent at 78°C-85°C to obtain a honokiol solution, rapidly pouring the honokiol solution into another container, standing at room temperature overnight, and centrifuging to collect a solid as honokiol crystal form A,
wherein the organic solvent is n-heptane, petroleum ether, cyclohexane, n-hexane, toluene, DCM, DMSO, NMP, trichloromethane, methanol/water, or ethanol/water;
method II: preparing honokiol crystal form A by gas-solid infiltration
placing honokiol in a container, then adding an organic solvent to another container, placing the opened container containing honokiol in the container containing the organic solvent, sealing and standing at room temperature, and centrifuging to collect a solid as honokiol crystal form A,
wherein the organic solvent is ethyl acetate, isopropyl acetate, methyl tert-butyl ether, trichloromethane, N-methylpyrrolidone, ethanol or acetonitrile;
method III: preparing honokiol crystal form A by gas-liquid infiltration
placing honokiol in a container, dissolving in a solvent and filtering to obtain a clear honokiol solution, adding an anti-solvent to another container, placing the opened container containing the clear honokiol solution in the container containing the anti-solvent, sealing and standing at room temperature, and centrifuging to collect a solid as honokiol crystal form A,
wherein the solvent is ethanol, tetrahydrofuran, trichloromethane, ethyl acetate or isopropanol, and the anti-solvent is n-hexane or water;
method IV: preparing honokiol crystal form A by suspension stirring at room temperature
placing honokiol in a container, adding an organic solvent to obtain a suspension, subjecting the obtained suspension to magnetic stirring at room temperature, and centrifuging to collect a solid as honokiol crystal form A,
wherein the organic solvent is methanol/water, acetonitrile/water, acetone/water, or dichloromethane/n-hexane;
method V: preparing honokiol crystal form A by suspension stirring at 40-60°C
placing honokiol in a container, adding an organic solvent to obtain a suspension, subjecting the obtained suspension to magnetic stirring at 40-60°C, and centrifuging to collect a solid as honokiol crystal form A,
wherein the organic solvent is n-heptane, Tween/n-heptane, trichloromethane/n-heptane, or isopropanol/water;
method VI: preparing honokiol crystal form A by stirring at temperature cycling
placing honokiol in a container, adding an organic solvent to obtain a suspension, subjecting the obtained suspension to magnetic stirring at a temperature cycling, and centrifuging to collect a solid as honokiol crystal form A,
wherein the temperature cycling comprises 2 cycles of 60°C→5°C, 0.1°C/min, and 5°C→60°C, 1.5°C/min, and preferably 2 cycles of 50°C→5°C, 0.1°C/min, and 5°C→50°C, 1.5°C/min,
wherein the organic solvent is n-heptane, ethanol/water, acetone/water, tetrahydrofuran/water, acetonitrile/water, ethyl acetate/n-heptane, methyl tert-butyl ether/n-hexane, or methyl isobutyl ketone/n-hexane;
method VII: preparing honokiol crystal form A by slow volatilization
placing honokiol in a container, dissolving in an organic solvent, filtering and taking the filtrate, sealing the container containing the filtrate with a sealing membrane, making small holes on the sealing membrane, standing at room temperature to slowly volatilize, and centrifuging to collect a solid as honokiol crystal form A,
wherein the organic solvent is trichloromethane, dichloromethane, methanol, isopropanol, or methyl isobutyl ketone; and
method VIII: preparing honokiol crystal form A by anti-solvent addition
placing honokiol in a container, dissolving in a solvent, adding an anti-solvent under magnetic stirring while adding dropwise, standing, and centrifuging to collect a solid as honokiol crystal form A,
wherein the solvent is ethanol, acetone, methyl isobutyl ketone, ethyl acetate, methyl tert-butyl ether, acetonitrile, dichloromethane or trichloromethane, and the anti-solvent is n-heptane or water.

4. A honokiol crystal form B, wherein an X-ray powder diffraction pattern of honokiol crystal form B comprises characteristic peaks at 2theta values of 6.15°±0.2°, 6.76°±0.2°, 8.96°±0.2° and 15.90°±0.2° under Cu-Kα radiation.

5. The honokiol crystal form B according to claim 4, wherein the X-ray powder diffraction pattern of honokiol crystal form B comprises characteristic peaks at 2theta values of 6.15°±0.2°, 6.76°±0.2°, 8.96°±0.2°, 15.90°±0.2°, 17.49°±0.2° and 18.55°±0.2° under Cu-Kα radiation.

6. A preparation method of honokiol crystal form B according to claim 4 or 5, comprising the following steps of
heating the honokiol crystal form A according to claim 1 or 2 to 80°C-82°C and then cooling to room temperature, thus obtaining a solid as honokiol crystal form B.

7. A honokiol crystal form C, wherein an X-ray powder diffraction pattern of honokiol crystal form C comprises characteristic peaks at 2theta values of 14.04°±0.2°, 15.84°±0.2°, 17.42°±0.2° and 19.48°±0.2° under Cu-Kα radiation.

8. The honokiol crystal form C according to claim 7, wherein the X-ray powder diffraction pattern of honokiol crystal form C comprises characteristic peaks at 2theta values of 14.04°±0.2°, 15.84°±0.2°, 17.42°±0.2°, 19.48°±0.2°, 21.26°±0.2°, 23.10°±0.2° and 24.06°±0.2° under Cu-Kα radiation.

9. A preparation method of honokiol crystal form C according to claim 7 or 8, comprising the following steps of:
(1) heating honokiol crystal form A according to claim 1 or 2 to a molten state at 83°C-100°C and stirring; and
(2) placing the melt obtained in step (1) rapidly at a quenching temperature of -20°C to -196°C to separate out a crystal as honokiol crystal form C.

10. The method according to claim 9, wherein the quenching temperature of the step (2) is -80°C to -196°C.

11. A honokiol amorphous form, wherein the honokiol amorphous form has an X-ray powder diffraction pattern as shown in FIG. 19.

12. A preparation method of the honokiol amorphous form according to claim 11, comprising the following steps of:
(1) dissolving honokiol crystal form A according to claim 1 or 2 with DMSO;
(2) adding anti-solvent water to the solution obtained in step (1) and stirring; and
(3) centrifuging, discarding the supernatant, standing at room temperature, and thus obtaining an oily matter as honokiol amorphous form.

13. A pharmaceutical composition or pharmaceutical preparation, comprising a therapeutically effective amount of the honokiol crystal form A according to claim 1 or 2, the honokiol crystal form B according to claim 4 or 5, the honokiol crystal form C according to claim 7 or 8, or the honokiol amorphous form according to claim 11, and pharmaceutical excipients.

14. A honokiol nano-liposome lyophilized powder, comprising a therapeutically effective amount of the honokiol crystal form A according to claim 1 or 2, the honokiol crystal form B according to claim 4 or 5, the honokiol crystal form C according to claim 7 or 8, or the honokiol amorphous form according to claim 11.

15. A preparation method of the honokiol nano-liposome lyophilized powder according to claim 14, comprising the following steps of
dissolving honokiol crystal form A, honokiol crystal form B, honokiol crystal form C or the honokiol amorphous form, phospholipid, phosphatidylethanolamine and cholesterol in absolute ethanol, injecting the solution that is completely dissolved into purified water, stirring, subjecting to rotary evaporation to remove ethanol, adding a freeze-dried excipient, and freeze-drying to obtain the honokiol nano-liposome lyophilized powder.

16. Use of the honokiol crystal form A according to claim 1 or 2, the honokiol crystal form B according to claim 4 or 5, the honokiol crystal form C according to claim 7 or 8, or the honokiol amorphous form according to claim 11 in the preparation of medicaments for treating tumors.

17. Use of the honokiol crystal form A according to claim 1 or 2, the honokiol crystal form B according to claim 4 or 5, the honokiol crystal form C according to claim 7 or 8, or the honokiol amorphous form according to claim 11 in the preparation of antimicrobial, antioxidant, anxiolytic, antidepressant or antithrombotic medicaments.
